# EUROPEAN PATENT SPECIFICATION

(11) **EP 1 393 732 B1**
(45) Date of publication and mention of the grant of the patent: **01.08.2007**
(21) Application number: 02724781.6
(22) Date of filing: 14.05.2002
(51) Int. Cl.: A61K 31/506, A61K 8/44, A61K 8/49, A61K 9/08, A61K 47/10, A61K 47/18, A61P 17/14, A61Q 7/00

(54) **MINOXIDIL-CONTAINING LIQUID COMPOSITION**
MINOXIDIL-ENTHALTENDE FLÜSSIGE ZUSAMMENSETZUNG
COMPOSITION LIQUIDE CONTENANT DU MINOXIDIL

(30) Priority: 15.05.2001 JP 2001145028
(43) Date of publication of application: 03.03.2004
(73) Proprietor: TAISHO PHARMACEUTICAL CO., LTD, Tokyo 170-8633 (JP)
(72) Inventor: Imamura, Koji, Taisho Pharmaceutical Co., Ltd., Toshima-ku, Tokyo 170-8633 (JP); Ochiai, Rumi, Taisho Pharmaceutical Co., Ltd., Toshima-ku, Tokyo 170-8633 (JP); Okajima, Takako, Taisho Pharmaceutical Co., Ltd., Toshima-ku, Tokyo 170-8633 (JP); Morioka, Susumu, Taisho Pharmaceutical Co., Ltd., Toshima-ku, Tokyo 170-8633 (JP)
(74) Representative: HOFFMANN EITLE
(86) International application number: PCT/JP2002/004657
(87) International publication number: WO 2002/092092

(56) References cited:
- EP-A1- 1 050 310
- WO-A-02/09664
- WO-A1-02/11698
- WO-A1-99/53923
- WO-A2-00/48559
- JP-A- 2002 029 935
- JP-A- 2002 173 449

## Description

### TECHNICAL FIELD

The present invention relates to a minoxidil-containing liquid composition with a preventive effect on color development by heat with time.

### BACKGROUND ART

Minoxidil is an ingredient for use as an external hair regrowth agent based on its hair regrowth effect.

However, minoxidil has such a drawback as easy color development with time when reserved in a solution state, sometimes resulting in a decrease in the commercial value. Color development mechanism of minoxidil-containing liquid preparation has not been completely clarified yet, but it seems that light and heat are taking part therein. Thus, in the commercialization of minoxidil-containing liquid preparation, it is the ordinary practice to use light-shielded containers to shut out any light influence.

JP-A-11-263727 discloses addition of an amino acid, particularly a sulfur-containing amino acid including taurine to a minoxidil-containing liquid preparation to prevent color development with time. The proposed method can prevent color development by light, but has no satisfactory effect on prevention of color development by heat with time.

When an ingredient generally easy to undergo color development is added to a liquid preparation, an antioxidant or a chelating-preventive agent or the like is further added thereto to prevent color development, but such a further addition has no satisfactory effect on prevention of a minoxidil-containing liquid preparation from color development by heat with time.

It seems that the simplest method for preventing a decrease in the commercial value is to provide a hair regrowth agent as colored from the beginning so that color development of the preparation, even if developed with time, may not be remarkable. However, the hair regrowth agent is used upon application to the skin of the head, so the preparation, as colored from the beginning, will stain clothing, bedclothes, etc., and thus is unpreferable.

### DISCLOSURE OF THE INVENTION

An object of the present invention is to prevent a minoxidil-containing liquid preparation from color development by heat.

As a result of extensive studies to solve the above-mentioned problems, the present inventors have found that color development of the liquid composition by heat can be unexpectedly prevented by adding arginine to a minoxidil-containing liquid preparation.

According to an aspect of the present invention, there is provided a liquid composition which comprises minoxidil and arginine contained in a liquid medium at a pH of 8 11. In another aspect of the present invention, there is provided an agent for preventing color development of a minoxidil-containing liquid preparation at a pH of 8 to 11 by heat, which comprises arginine as an effective ingredient.

In other aspect of the present invention, there is provided a method for preventing color development of a minoxidil-containing liquid composition by heat, characterized by adding arginine to a minoxidil-containing liquid preparation at a pH of 8 to 11.

In the present invention, the term "liquid composition" herein used is meant to cover any liquid state form containing minoxidil and arginine, where the form may be a solution state form or a suspension state form.

In the present invention, the term "arginine" herein used is the generic term covering free state arginine and its salts such as sodium salt, potassium salt, hydrochloride, etc. Arginine herein used includes D-, Land DL-isomers, more preferably L-isomers and salts thereof.

In the present invention, the term "color development by heat" herein used means color development by an influence of external heat, including, for example, by temperature elevation due to changes in surroundings, climatic temperature, etc. while preserved.

### BEST MODE FOR CARRYING OUT THE INVENTION

The present liquid composition contains minoxidil and arginine as essential ingredients and the pH of the liquid composition is in the range of 8 to 11.

Concentration of minoxidil as one ingredient of the present liquid composition is preferably 0.1 to 6% by mass on the basis of total mass of the liquid composition. Below 0.1% by mass the effect of minoxidil as a hair regrowth agent will be lower, whereas above 6% by mass a preparation design will be hard to make.

A proportion of arginine to be added as another ingredient of the present liquid composition is preferably 0.003 to 0.6 part by mass, more preferably 0.003 to 0.4 part by mass, most preferably 0.03 to 0.4 part by mass on the basis of one part by mass of minoxidil from the viewpoints of arginine solubility and effect on prevention of color development.

Liquid medium for use in the present liquid composition is preferably an organic solvent containing water. Arginine as one ingredient of the present liquid composition is sparingly soluble in an organic solvent, whereas minoxidil as another ingredient is sparingly soluble in water. To bring these two ingredients into a solution state, an organic solvent containing water is thus preferable. Preferable water content of the liquid medium, though dependent on proportions of minoxidil and arginine contained in the liquid composition, is usually 10 to 50 V/V%. The organic solvent to be used must be a solvent uniformly miscible with water and having no adverse effect on the skin, even if used as a skin external preparation. On this ground, for example, an alcohol which is in a liquid state at ordinary temperatures is a preferable organic solvent. The term "ordinary temperatures" herein used means temperatures in a range of 10°C to 30°C, and the term "alcohol" herein used is the generic term covering monohydric, dihydric and polyhydric alcohols. Preferable examples of monohydric alcohols which are in a liquid state at ordinary temperatures include monohydric alcohols having 1 to 6 carbon atoms, such as ethanol, propanol, isopropanol, etc. Preferable examples of dihydric and polyhydric alcohols which are in a liquid state at ordinary temperatures include alkylene glycols, particularly such as ethylene glycol, propylene glycol, 1,3-butylene glycol, glycerin, dipropylene glycol, macrogol 300, macrogol 400, etc.

Among the above-exemplified alcohols which are in a liquid state at ordinary temperatures, at least one member selected from the group consisting of ethanol, propylene glycol, 1,3-butylene glycol, glycerin and dipropylene glycol is particularly preferable.

To keep minoxidil stable in the present liquid composition, the liquid composition is kept at a pH of 8 to 11.

The present liquid composition can be prepared by the ordinary method for preparing liquid compositions, but to mix arginine with an organic solvent, it is preferable to add a solution of arginine in water, as prepared beforehand, to the organic solvent, because it takes much time to dissolve the arginine into the organic solvent containing water by direct addition of arginine thereto.

The present liquid composition can contain ingredients usually used in the liquid preparation for external use (a solubilizing agent, a thickening agent, an absorption promoter, a pH controlling agent, etc.)

The present liquid composition can be used in various forms so far usually used as hair regrowth agents such as hair tonic, hair liquid, hair lotion, gel, cream, aerosol, etc.

The present invention will be described in detail below, referring to Examples and Test Examples, which shall not be construed as restrictive of the scope of the present invention. Various changes and modifications can be made within the scope of the present invention by those skilled in the art, and such changes and modifications shall be construed as inclusive within the scope of the present invention.

### Examples

### Examples 1 to 5

Liquid compositions of Examples 1 to 5 and Comparative Example 1 were prepared according to formulations given in Table 1. That is, minoxidil, arginine, 1,3-butylene glycol and ethanol were mixed together, and then purified water was added thereto in an amount to make total volume 100 ml, followed by stirring and dissolution to prepare liquid compositions. In Example 5, arginine was not completely dissolved until the second week after the preparation and was found to be in a suspension state.

### Comparative Example 1

A comparative liquid preparation was made according to the same formulation as in Example 1 except that no arginine was used.

**Table 1**

| Ingredient | Ex. 1 | Ex. 2 | Ex. 3 | Ex. 4 | Ex. 5 | Comp. Ex. 1 |
|---|---|---|---|---|---|---|
| Minoxidil | 3 g | 3 g | 3 g | 3 g | 3 g | 3 g |
| L-arginine | 0.01 g | 0.1 g | 0.3 g | 0.5 g | 1.0 g | - |
| 1,3-butylene glycol | 5 g | 5 g | 5 g | 5 g | 5 g | 5 g |
| 95% ethanol | 64.34 g | 64.34 g | 64.34 g | 64.34 g | 64.34 g | 64.34 g |
| Purified water | To make total volume 100 ml | To make total volume 100 ml | To make total volume 100 ml | To make total volume 100 ml | To make total volume 100 ml | To make total volume 100 ml |
| pH | 9.45 | 10.34 | 10.86 | 10.62 | not determined | 8.50 |

### Test Example 1

Liquid preparations obtained in Example 1 and Comparative Example 1 were filled into light-shielded plastic containers, respectively, and preserved at 40°C for 12 weeks. Degree of color development of the liquid preparations were periodically determined by visual observation (according to the general rules of the Japanese Pharmacopoeia) and absorbancy at wavelength of 420 nm (by a spectrophotometer, cell length: 1 cm). The results are shown in Table 2.

**Table 2**

| | Just after preparation | 1 week thereafter | 2 weeks thereafter | 3 weeks thereafter | 6 weeks thereafter | 9 weeks thereafter | 12 weeks thereafter | Visual observation after 12 weeks |
|---|---|---|---|---|---|---|---|---|
| Ex. 1 | 0.001 | 0.015 | 0.033 | 0.044 | 0.084 | 0.111 | 0.142 | Light yellow transparent |
| Ex. 2 | 0.001 | 0.012 | 0.018 | 0.024 | 0.043 | 0.060 | 0.081 | Slightly yellowish transparent |
| Ex. 3 | 0.002 | 0.012 | 0.017 | 0.024 | 0.049 | 0.073 | 0.106 | Slightly yellowish transparent |
| Ex. 4 | 0.002 | 0.013 | 0.020 | 0.025 | 0.052 | 0.079 | 0.115 | Slightly yellowish transparent |
| Ex. 5 | - | - | 0.017 | 0.018 | 0.037 | 0.060 | 0.091 | Slightly yellowish transparent |
| Comp. Ex. 1 | 0.001 | 0.032 | 0.080 | 0.109 | 0.198 | 0.257 | 0.317 | Dark yellow transparent |

As is apparent from the above Table, it was found that addition of arginine could prevent color development of the preparations.

### Examples 6 to 9

To investigate relationship between pH change and color development, some of L-arginine-containing liquid preparations obtained in the same manner as in Example 1 were subjected to pH adjustment with hydrochloric acid, thereby obtaining preparations with the pH fitting that of L-arginine-free formulation.

### Comparative Example 2

Comparative liquid preparation was prepared according to the same formulation as in Example 6 except that no arginine was used.

**Table 3**

| Ingredient | Ex. 6 | Ex. 7 | Ex. 8 | Ex. 9 | Comp. Ex. 2 |
|---|---|---|---|---|---|
| Minoxidil | 3 g | 3 g | 3 g | 3 g | 3 g |
| L-arginine | 0.1 g | 0.1 g | 0.3 g | 0.3 g | - |
| 1,3-butylene glycol | 5 g | 5 g | 5 g | 5 g | 5 g |
| Hydrochloric acid | - | ○ | - | ○ | - |
| 95% ethanol | 64.34 g | 64.34 g | 64.34 g | 64.34 g | 64.34 g |
| Purified water | To make total volume 100 ml | To make total volume 100 ml | To make total volume 100 ml | To make total volume 100 ml | To make total volume 100 ml |
| pH | 10.37 | 8.46 | 10.77 | 8.45 | 8.44 |

### Test Example 2

Liquid preparations obtained in Examples 6 to 9 and Comparative Example 2 were preserved at 40°C for 2 weeks, and color development was determined by absorbancy at 420 nm and visual observation in the same manner as in Test Example 1. The results are shown in Table 4.

**Table 4**

| Sample | Just after preparation | 1 week thereafter | 2 weeks thereafter | |
|---|---|---|---|---|
| Ex. 6 | 0.005 | 0.017 | 0.028 | colorless transparent |
| Ex. 7 | 0.011 | 0.035 | 0.067 | Slightly yellowish transparent |
| Ex. 8 | 0.004 | 0.016 | 0.031 | colorless transparent |
| Ex. 9 | 0.012 | 0.033 | 0.062 | Slightly yellowish transparent |
| Comp. Ex. 2 | 0.003 | 0.071 | 0.180 | Light yellow transparent |

As is apparent from the above Table, it was found that the effect of the present invention on prevention of color development was not by mere pH changes, but by arginine.

### INDUSTRIAL APPLICABILITY

The present invention can prevent color development of minoxidil-containing liquid composition by heat with time, and thus can provide a minoxidil-containing liquid composition with a good preservation stability and improved commercial value.

## Claims

1. A liquid composition which comprises minoxidil and arginine contained in a liquid medium, wherein the proportion of arginine is 0.003 to 0.6 part by mass on the basis of one part by mass of minoxidil, and wherein the proportion of minoxidil is 0.1 to 6% by mass on the basis of the total mass of the liquid composition, the pH of the liquid composition being in the range of 8 to 11.

2. A liquid composition according to Claim 1, wherein the liquid medium is an organic solvent containing water.

3. A liquid composition according to Claim 2, wherein the liquid medium has a water content of 10 to 50 V/V%.

4. A liquid composition according to Claim 2 or 3, wherein the organic solvent containing water is a liquid mixture of water and an alcohol which is in a liquid state at ordinary temperatures.

5. A liquid composition according to Claim 4, wherein the alcohol which is in a liquid state at ordinary temperatures is at least on member selected from the group consisting of ethanol, propylene glycol, 1,3-butylene glycol, glycerine and dipropylene glycol.

6. A liquid composition according to any one of Claims 1-5, which is a hair regrowth agent.

7. Use of arginine for the prevention of color devolpement by heat of a minoxidil-containing liquid preparation at a pH of 8 to 11.

8. A method for preventing color development of a minoxidil-containing liquid composition by heat, **characterized by** adding arginine to a minoxidil-containing liquid preparation at a pH of 8 to 11.

## Patentansprüche

1. Flüssige Zusammensetzung, die Minoxidil und Arginin, enthalten in einem flüssigen Medium, umfaßt, worin der Anteil von Arginin 0,003 bis 0,6 Massenteile, bezogen auf ein Massenteil Minoxidil, beträgt, und worin der Anteil von Minoxidil 0,1 bis 6 Massen%, bezogen auf die Gesamtmasse der flüssigen Zusammensetzung, beträgt, wobei der pH-Wert der flüssigen Zusammensetzung im Bereich von 8 bis 11 liegt.

2. Flüssige Zusammensetzung gemäß Anspruch 1, worin das flüssige Medium ein organisches Lösungsmittel ist, das Wasser enthält.

3. Flüssige Zusammensetzung gemäß Anspruch 2, worin das flüssige Medium einen Wassergehalt von 10 bis 50 V/V% aufweist.

4. Flüssige Zusammensetzung gemäß Anspruch 2 oder 3, worin das organische Lösungsmittel, das Wasser enthält, eine flüssige Mischung aus Wasser und einem Alkohol ist, der bei normalen Temperaturen im flüssigen Zustand vorliegt.

5. Flüssige Zusammensetzung gemäß Anspruch 4, worin der Alkohol, der bei normalen Temperaturen in einem flüssigen Zustand vorliegt, zumindest ein Mitglied aus der Gruppe ist, die aus Ethanol, Propylenglycol, 1,3-Butylenglycol, Glycerin und Dipropylenglycol besteht.

6. Flüssige Zusammensetzung gemäß irgendeinem der Ansprüche 1 bis 5, das ein Haarwachstumsmittel ist.

7. Verwendung von Arginin zur Vorbeugung von Farbentwicklung durch Wärme einer Minoxidil-haltigen flüssigen Zubereitung mit einem pH-Wert von 8 bis 11.

8. Verfahren zur Vorbeugung von Farbentwicklung einer Minoxidil-haltigen flüssigen Zusammensetzung durch Wärme, **dadurch gekennzeichnet, daß** Arginin zu einer Minoxidil-haltigen flüssigen Zubereitung bei einem pH-Wert von 8 bis 11 zugegeben wird.

## Revendications

1. Composition liquide comprenant du minoxidil et de l'arginine contenus dans un milieu liquide, dans laquelle la proportion d'arginine est de 0,003 à 0,6 partie en masse sur la base d'une partie en masse de minoxidil, et dans laquelle la proportion de minoxidil est de 0,1 à 6% en masse sur la base de la masse totale de la composition liquide, le pH de la composition liquide allant de 8 à 11.

2. Composition liquide selon la revendication 1, dans laquelle le milieu liquide est un solvant organique contenant de l'eau.

3. Composition liquide selon la revendication 2, dans laquelle le milieu liquide présente une teneur en eau de 10 à 50 % V/V.

4. Composition liquide selon la revendication 2 ou 3, dans laquelle le solvant organique contenant de l'eau est un mélange liquide d'eau et d'un alcool qui se trouve à l'état liquide à des températures ordinaires.

5. Composition liquide selon la revendication 4, dans laquelle l'alcool qui se trouve à l'état liquide à des températures ordinaires est au moins un élément choisi dans le groupe contenant l'éthanol, le propylène glycol, le 1,3-butylène glycol, la glycérine et le dipropylène glycol.

6. Composition liquide selon l'une quelconque des revendications 1 à 5, qui est un agent de repousse des cheveux.

7. Utilisation de l'arginine pour prévenir le développement de la couleur par la chaleur d'une préparation liquide contenant du minoxidil à un pH de 8 à 11.

8. Procédé destiné à empêcher le développement de la couleur d'une composition liquide contenant du minoxidil par la chaleur, **caractérisé par** l'ajout d'arginine à une préparation liquide contenant du minoxidil à un pH de 8 à 11.
